Europäisches Patentamt

European Patent Office

Office européen des brevets

(19)

(11) Veröffentlichungsnummer : **0 412 466 B1**

## (12) EUROPÄISCHE PATENTSCHRIFT

(45) Veröffentlichungstag der Patentschrift :
**02.02.94 Patentblatt 94/05**

(51) Int. Cl.$^5$ : **A61K 35/16**

(21) Anmeldenummer : **90115005.2**

(22) Anmeldetag : **04.08.90**

(54) **Verfahren zur Herstellung eines stabilen Faktors VIII.**

(30) Priorität : **07.08.89 DE 3926034**

(43) Veröffentlichungstag der Anmeldung :
**13.02.91 Patentblatt 91/07**

(45) Bekanntmachung des Hinweises auf die
Patenterteilung :
**02.02.94 Patentblatt 94/05**

(84) Benannte Vertragsstaaten :
**AT BE CH DE DK ES FR GB GR IT LI LU NL SE**

(56) Entgegenhaltungen :
**EP-A- 0 032 655
EP-A- 0 104 356
EP-A- 0 173 242
EP-A- 0 221 566
WO-A-89/12065
GB-A- 1 178 958**

(73) Patentinhaber : **BEHRINGWERKE
Aktiengesellschaft
Postfach 1140
D-35001 Marburg (DE)**

(72) Erfinder : **Heimburger, Norbert, Dr. Prof.
Sonnenhang 10
D-3550 Marburg (DE)**
Erfinder : **Wellner, Klaus
Zum Kalkberg 4
D-3550 Marburg (DE)**
Erfinder : **Wenz, Karl-Heinz
Talstrasse 10
D-3556 Argenstein (DE)**
Erfinder : **Kumpe, Gerhardt
Perbaler Weg 11
D-3552 Wetter (DE)**

## Beschreibung

Die Erfindung betrifft ein Verfahren zur Herstellung eines pasteurisierten Faktor VIII-Konzentrates mit hoher spezifischer Aktivität und Stabilität, worin aus der Faktor VIII enthaltenden Lösung Verunreinigungen durch mindestens zweimalige Adsorption mit $Al(OH)_3$ und eine Adsorption an einem Anionenaustauscher und/oder $Ca_3(PO_4)_2$ adsorbiert werden.

Die plasmatische Gerinnung ist ein enzymatischer Prozeß; die daran beteiligten Faktoren sind Proteine, vorwiegend mit der Eigenschaft von Proteasen, die in Form ihrer inaktiven Vorstufen im Blut zirkulieren und erst beim Kontakt mit benetzbaren Oberflächen oder aber bei Verletzungen aktiviert werden. Neben dem Thrombin greifen auch andere Gerinnungsproteasen, z.B. die Faktoren Xa und IXa, den Faktor VIII an und können ihn inaktivieren. Besondere Bedeutung kommt auch dem Protein C zu, das auch den Faktor Va inaktivieren kann. Die Faktoren V und VIII sind die labilsten Gerinnungsfaktoren. Vom Augenblick der Blutentnahme an beginnen sie zu zerfallen. Dazu trägt noch bei, daß sie durch $Ca^{2+}$ stabilisiert werden und die Blutentnahme mit Komplexbildnern wie Citrat und EDTA erfolgt. Diese Erkenntnisse sind in Übereinstimmung mit der Beobachtung, daß man im Kryopräzipitat aus Humanplasma, das weltweit als Ausgangsmaterial für die Faktor VIII-Gewinnung verwendet wird, nur noch 40 bis 60 % der Faktor VIII-Aktivität aus Humanplasma findet und daß davon bei der Reinigung von Faktor VIII nur noch 10 bis 20 % übrigbleiben. Das ist ein großes Handicap für die Versorgung der Hämophilen; denn zum einen ist der Rohstoff Blut nicht nur kostbar und steht nur sehr begrenzt zur Verfügung, zum anderen wirken sich die geringen Ausbeuten auf den Preis aus. Das gilt ganz besonders seit Einführung von zusätzlichen Arbeitsschritten, die eine Virusinaktivierung bei der Faktor VIII-Herstellung gewährleisten.

Für einen mit konventionellen Methoden hergestellten Faktor VIII geben Fay et al. (Proc.Natl.Acad.Sci., USA, 79, 7200, 1982) eine Ausbeute von 12,7 % an. Auf einen nahezu identischen Wert kommen Zimmerman und Fulcher mit den modernen Methoden der Immunaffinitätschromatographie (EP-A-0 083 483).

Viele Autoren haben beschrieben, daß sich der Faktor VIII fest an DEAE-Austauscher und ECTEOLA bindet, zwar mit Halogeniden wieder verdrängt werden kann, aber innerhalb von 24 Std. seine Aktivität verliert (S. van Creveld et al., Thromb.Diathes. VI (2/3), 282, 1961; R. Baugh et al., Biochim.Biophys.Acta 371, 360, 1974), ohne daß eine Lösung des Problems angeboten wurde, da Zusätze von Proteinase-Inhibitoren wie PMSF (Phenylmethansulfonyl-Fluorid) und Benzamidin nicht geeignet sind, um ein Humanpräparat herzustellen.

Bedingt durch die beschriebene Situation bestand ein dringender Bedarf an Maßnahmen, die einen Zerfall des Faktor VIII während der Aufarbeitung bzw. Reinigung verhindern und möglichst frühzeitig, d.h. gleich zu Beginn der Isolierung oder der Reinigung eingesetzt werden können. Als einen frühen Zeitpunkt wurde die Weiterverarbeitung von Kryopräzipität gewählt, das, ausgehend von Plasma, durch eine sogenannte Kryopräzipitation gewonnen und auch als Rohstoff gehandelt wird.

In der Regel werden nach dem Stand der Technik die gelösten Kryopräzipitate mit $Al(OH)_3$ behandelt, um Spurenverunreinigungen von Prothrombinfaktoren (Faktor II, VII, IX, X) zu eliminieren. Damit will man eine Aktivierung dieser Faktoren, die den Faktor VIII während der Reinigung abbauen könnten, verhindern. Überraschenderweise fanden wir, daß eine einmalige Adsorption einer frischen Lösung von Kryopräzipitat mit $Al(OH)_3$ nicht ausreicht, um die Prothrombinfaktoren und andere Proteasen zu entfernen; denn auch noch nach der Adsorption konnte eine deutliche amidolytische Aktivität im Überstand der adsorbierten Lösung nachgewiesen werden, am empfindlichsten mit dem F Xa-Test, obwohl diese Aktivität mit F Xa nicht identisch zu sein scheint.

Überraschenderweise wurde gefunden, daß durch Vorbehandlung eines Kryopräzipitats, zum Beispiel durch die mehrfache Adsorption an $Al(OH)_3$, $Ca_3(PO_4)_2$ oder Anionenaustauscher oder auch Misch-Adsorptionen, ein stabiler Faktor VIII erhalten werden kann, der sich gut und verlustarm handhaben läßt. Als Kriterium dafür wird vom Endprodukt die amidolytische Aktivität, die Ausbeute, die spezifische Aktivität und die Belastbarkeit bei Raumtemperatur bestimmt.

Gegenstand der Erfindung sind daher Maßnahmen, die verhindern, wenn man sie auf ein frisch gelöstes Kryopräzipitat anwendet, daß ein großer Anteil der Faktor VIII-Aktivität während der Reinigung und Pasteurisierung verlorengeht, und die zu einem Endprodukt führen, das bei guter Ausbeute durch eine hohe spezifische Aktivität, Nativität und Stabilität gekennzeichnet ist.

Gegenstand der Erfindung ist besonders ein Verfahren entsprechend den Ansprüchen zur Behandlung von F VIII-haltigen Plasma-Fraktionen, wie sie üblicherweise für die F VIII-Herstellung verwendet werden, vorzugsweise von gelöstem Kryopräzipitat oder einer Cohn-Fraktion I, dadurch gekennzeichnet, daß sie mit Adsorbentien behandelt werden, die amidolytisch/proteolytisch wirksame Enzyme und Proenzyme, die den F VIII abbauen, binden, wonach mit an sich bekannten Methoden ein stabiler und gegebenenfalls pasteurisierter Faktor VIII mit guter Ausbeute erhalten werden kann.

Gegenstand der Erfindung ist besonders ein Verfahren zur Herstellung von Faktor VIII mit hoher spezifischer Aktivität und Stabilität, dadurch gekennzeichnet, daß man eine Faktor VIII-haltige Lösung wie die von Kryopräzipitat mindestens zwei Adsorptionen an $Al(OH)_3$ und einer Adsorption an einem Anionenaustauscher oder $Ca_3(PO_4)_2$ unterwirft, bevor man aus der so vorbehandelten Lösung mit an sich bekannten Methoden den Faktor VIII gewinnt.

Solche bekannten Methoden sind beispielsweise in EP-A-0 018 561 oder EP-A-0 173 242 beschrieben.

Vorzugsweise wird das F VIII-haltige Ausgangsmaterial mit einer Mischung von mindestens zwei verschiedenen Adsorbentien adsorbiert. Günstig ist eine Behandlung mit $Ca_3(PO_4)_2$ oder aber mit einem basischen Anionenaustauscher mit hydrophiler Matrix beispielsweise auf Polysaccharidbasis wie Zellulose oder vernetzte Polysaccharide und funktionellen Gruppierungen, wie sie z.B. kennzeichnend für DEAE-[R]Sephadex, ECTEOLA oder QAE-[R]Sephadex (QAE) sind.

Die Auswahl des Typs und der Menge an Adsorbens (in der Regel 1 - 3 % W/V) erfolgt unter dem Gesichtspunkt, daß Zymogene und Enzyme, vorwiegend Proteinasen, wie sie mit dem F VIII vergesellschaftet vorkommen können, adsorbiert und entfernt werden, jedoch nicht F VIII. Als Indikator dafür wird die amidolytische Aktivität des Endproduktes bestimmt, bevorzugt unter Verwendung von chromogenen Peptiden, wie sie für die F Xa-Bestimmung eingesetzt werden und handelsüblich sind. Das F VIII-Endprodukt sollte praktisch frei davon sein.

Wenn mehrere Adsorbentien verwendet werden, können sie einzeln zugegeben und auch abgetrennt werden, vorzugsweise werden sie jedoch im Abstand von 1 min bis 30 min nacheinander zugegeben und zusammen abgetrennt. Eine bevorzugte Ausführungsform sieht die Abzentrifugation aller zugesetzten Adsorbentien nach Adsorptionszeiten von 1 - 30 min vor.

Die Behandlung einer Lösung eines Kryopräzipitates (Kryolösung) kann vorteilhaft bei einem pH-Wert von 6,5 - 7,5 und physiologischer Ionenstärke (10-15 mS) durchgeführt werden, bevorzugt in einem Medium, das keine Citrat-Ionen oder andere Fänger von zweiwertigen Metallionen enthält, am besten zunächst mit $Al(OH)_3$, aber auch zusammen mit $Ca_3(PO_4)_2$ und/oder DEAE-Austauscher und/oder ECTEOLA und/oder QAE. Voraussetzung für die Wirksamkeit der Behandlung ist, daß man die Adsorbentien zeitlich nacheinander zusetzt. Die Abtrennung durch Zentrifugation kann in einem Schritt erfolgen.

Die Ergebnisse solcher Adsorptionen vorwiegend mit Mischungen von Adsorbentien sind in der Tabelle 1 zusammengestellt. Sie lassen sich wie folgt zusammenfassen:

Die amidolytische Aktivität, die auch nach der zweiten $Al(OH)_3$-Adsorption noch in der Kryolösung verbleibt und im Endprodukt nachgewiesen werden kann, ist drastisch nur durch eine weitere nachfolgende Adsorption mit $Ca_3(PO_4)_2$ und/oder ECTEOLA und/oder QAE oder einen anderen basischen Ionenaustauscher zu senken; auch eine Mischung mehrerer Ionenaustauscher ist wirksam.

Überraschenderweise entsteht kein Verlust an Faktor VIII-Aktivität durch die Behandlung, wenn nicht eine bestimmte Menge an Adsorbens überschritten wird. Diese liegt für $Ca_3(PO_4)_2$ bei 10 g und für QAE und ECTEOLA bei ca. 20 bis 30 g angefeuchtetem Austauscher pro Liter Kryolösung. Eine solche Losung wird üblicherweise durch Lösen von 1 kg Kryopräzipitat in 3 l Pufferlösung erhalten.

**Tabelle 1**

Vorbehandlung einer Lösung von Kryopräzipitat mit Adsorbentien und basischen Ionenaustauschern: Einfluß auf Ausbeute, spezifische Aktivität und Stabilität von F VIII hergestellt nach EP-A 0 173 242 oder DE 34 32 083 A1

| Versuch-Nr. | Vorbehandlung mit Adsorbentien | Amidolyt. Aktivität* $OD_{405nm}$/min x $10^{-3}$ | Spez. Aktivität* (IE/mg) | Ver-fah-rens-aus-beu-te(%)* | Verlust an F VIII Akti-vität während der Dialyse *(20 h) (%) | Verlust an F VIII-Aktivität während Lagerung bei Raumtemperatur (20°C), angegeben in F VIII-Aktivität nach Dialyse(=100%) | | |
|---|---|---|---|---|---|---|---|---|
| | | | | | | 24 h | 48 h | 96 h |
| 1 | Al(OH)$_3$,1x | 31,5 | 56 | 48 | 19 | 29 | 70 | 98 |
| 2 | Al(OH)$_3$,2x | 22,0 | 75 | 44 | 5 | 12 | 23 | 37 |
| 3 | Al(OH)$_3$,2x ECTEOLA | 3,65 | 159 | 54 | 0,3 | 4 | 9 | 24 |
| 4 | Al(OH)$_3$,2x QAE | 1,85 | 144 | 54 | 8 | 5 | 7 | 23 |
| 5 | Al(OH)$_3$,2x QAE,ECTEOLA | 0,87 | 215 | 53 | 0 | 0 | 3 | 19 |
| 6 | Al(OH)$_3$,2x QAE,Ca$_3$(PO$_4$)$_2$ | 7,27 | 184 | 55 | 1 | 10 | 36 | 63 |

*bestimmt am Endprodukt

1 = Stand der Technik

Durch Vorbehandlung der Kryolösung mit Adsorbentien und Anionenaustauschern entfernt man zusätzlich zur amidolytischen Aktivität auch unspezifische Proteine. Dadurch werden sowohl die Ausbeute an Faktor VIII als auch die spezifische Aktivität angehoben. Die Vorbehandlung der frischen Kryolösung mit Adsorbentien und Ionenaustauschern hat weiterhin den Vorteil, daß sie zu einem stabilen Zwischenprodukt führt, das sich nahezu verlustfrei in wässriger Lösung bei 60 °C pasteurisieren und besser handhaben läßt. Sie führt zu einem stabilen Endprodukt, das sich ohne größere Einbußen an Aktivität dialysieren, filtrieren und lagern läßt.

Am wirksamsten war die Vorbehandlung der Kryolösung mit ECTEOLA, QAE-$^R$Sephadex, DEAE-$^R$Sephadex und einer Mischung dieser Anionenaustauscher in Verbindung mit einer vorausgegangenen Al(OH)$_3$-Adsorption. Bemerkenswert ist das Ergebnis der Mischadsorption mit QAE-$^R$Sephadex, Ca$_3$(PO$_4$)$_2$, weil sie mit hoher Ausbeute zu einem Produkt guter, spezifischer Aktivität und ausreichender Stabilität führt; denn eine Dialyse über 20 Std. bei 20°C und eine Lagerung über 24 Std. bei 20°C sind hohe Belastungen und der Aktivitätsverlust betrug nur 11 % insgesamt. Es ist noch zu vermerken, daß so stabile und gleichzeitig so eiweißarme F VIII-Lösungen, wie sie durch spezifische Aktivitäten von ca. 100 E/mg gekennzeichnet sind, bisher nicht beschrieben worden sind. Die Belastung über 4 Tage bei Raumtemperatur ist als Provokationstest zu betrachten, um auch letzte Spuren von Enzymverunreinigungen zu erkennen, die den Faktor VIII zerstören.

Es kann festgestellt werden, daß der Verlust an F VIII-Aktivität während der Lagerung auf Verunreinigungen der F VIII-Konzentrate mit Proteasen zurückzuführen ist und daß dieser mit der amidolytischen Restaktivität im Endprodukt korreliert (s. Tabelle 1).

Eine einmal mit Al(OH)$_3$ adsorbierte Probe enthält bereits nach Dialyse Spaltprodukte, entsprechend einer Molekularmasse von 50 bis 40 kDa, und während der Lagerung zerfällt sie praktisch vollständig in noch kleinere Untereinheiten (kleiner 40 kDa). Im Gegensatz dazu ist die mit zweimal Al(OH)$_3$ plus QAE und ECTEOLA behandelte Probe über 48 Stunden stabil. Das ist in guter Übereinstimmung mit den während der Lagerung gemessenen F VIII-Aktivitäten und auf die niedrige amidolytische Aktivität dieser Probe zurückzuführen.

Gegenstand der Erfindung ist ein hochgereinigtes Faktor VIII-Konzentrat mit klinisch guter Recovery und Halbwertszeit und ein Verfahren zu seiner Herstellung und Pasteurisierung mit guter Ausbeute und Stabilität, gekennzeichnet dadurch, daß der Rohstoff, speziell Kryopräzipitat, nach Lösen mit Adsorptionsmitteln, wie Al(OH)$_3$, Ca$_3$(PO$_4$)$_2$ und basischen Ionenaustauschern (DEAE, QAE, ECTEOLA) so lange behandelt wird, bis die Lösung, bestimmt am Endprodukt, frei von amidolytischer Aktivität ist.

Diese Bestimmung ist möglich mit dem Substrat S-2222 der Fa. Kabi (Bz-Ile-Glu-Gly-Arg-pNA), wie es für die Faktor X-Bestimmung verwendet wird; ebenso geeignet ist das BCP 200, Z-D-Leu-Gly-Arg-MNA der Behringwerke AG oder das $^R$Chromozym TH von Boehringer Mannheim GmbH.

Faktor VIII-Bestimmung

Die Bestimmung des Faktors VIII erfolgt beispielsweise nach folgendem Verfahren:

1 Teil, z. B. 0,1 ml partielles Thromboplastin, z. B. hergestellt nach der Patentanmeldung P 23 16 430.9-52 (Ma 160) wird mit einem Teil Faktor VIII-Mangelplasma und einem Teil verdünnten Normalplasma vermischt. Diese Mischung wird 6 min bei 37° C gehalten. Nach Zusatz von einem Teil einer auf 37° C vorgewärmten 0,025 molaren Calciumchlorid-Lösung wird die Zeit bestimmt, die vom Zusatz der Calciumchlorid-Losung bis zum Auftreten eines Gerinnsels verstreicht. Zur quantitativen Aussage wird die aus der Faktor VIII enthaltenden Lösung sich ergebende Gerinnungszeit unter Bezugnahme auf eine mit einer Normalplasma-Verdünnungsreihe erzielten Eichkurve abgelesen. 1 Internationale Einheit (= 1 IE) Faktor VIII entspricht der Faktor VIII-Aktivität von 1 ml Normalplasma.

Bestimmung der amidolytischen Aktivität

Diese kann beispielsweise nach dem Prinzip einer Faktor Xa-Bestimmung durchgeführt werden.
Bestimmungsansatz:
100 µl Probe + 500 µl Pufferlosung, 50 mmol/l Tris, 150 mmol/l NaCl, pH 8.2 + 100 µl Substrat, BCP 200, 3 mmol/l oder S-2222, 3 mmol/l

5 min bei 37° C vorinkubieren und anschließend die Umsetzung des Substrates über 20 min bei 37° C und 405 nm messen, Bewertung der amidolytischen Aktivität nach Delta OD/min.

Maßnahmen zur Stabilisierung von Faktor VIII können weitgehend unabhängig vom Ausgangsmaterial auf alle bisher beschriebenen Herstellungsverfahren angewandt werden, z. B. gemäß DE 34 32 083 bzw. EP 0 018 561 oder DE 34 32 083 bzw. EP 0 173 242 wie nachfolgend beispielhaft belegt wird. Die nachfolgend verwendeten Puffer sind nach den Beispielen beschrieben.

Beispiel 1

- **Ausgangsmaterial:** 1 kg Kryopräzipitat wurde mit 3 l Lösepuffer bei + 37° C gelöst und anschließend mit
- **1 x 8 % (v/v) Al(OH)$_3$** behandelt: 4 l Lösung wurden mit 320 ml Al(OH)$_3$ 1%ig 15 min lang bei + 25° C adsorbiert. Das Adsorptionsmittel wurde 15 min bei 3000 UpM abzentrifugiert und der Überstand der adsorbierten Kryolösung mit Stabilisatoren versetzt.

- **Stabilisieren und Erhitzen:**
Dazu wurden die 4040 ml des $Al(OH)_3$-Überstandes durch Zugabe von 4040 g Saccharose auf eine Endkonzentration entsprechend 100 % (w/v) gebracht; anschließend wurden 545,4 g Glycin entsprechend 1,8 mol/l zugesetzt und die $CaCl_2$-Konzentration auf 5 mmol/l eingestellt. Die stabilisierte Lösung wurde mit 2,5 N NaOH auf pH 7,3 eingestellt und dann 10 Std. bei + 60° C erhitzt. Vor und nach Erhitzen wurde die F VIII:C-Aktivität bestimmt, und zwar mit 2,0 E/ml bzw. 1,6 E/ml.
- **Vorbereitung zur DEAE-Adsorption** gemäß DE 34 32 083: Die erhitzte Kryolösung (6868 ml) wurde mit 6868 ml Verdünnungspuffer verdünnt und mit 2 N Essigsäure auf pH 5,5 eingestellt.
- **Adsorption an DEAE-RSepharose CL-6B im Batchverfahren:** 13736 ml verdünnte, pasteurisierte Kryolösung wurden mit 300 ml äquilibrierter DEAE-RSepharose CL-6B 4 Std. bei Raumtemperatur adsorbiert und anschließend die Sepharose abgetrennt.
- **Waschen der RSepharose und Überführen in eine Säule:** Die mit F VIII beladene Sepharose wurde auf einer Nutsche vorgewaschen, anschließend in eine Saule (∅ 7,2 cm) überführt und mit 3 l Waschpuffer behandelt.
- **Elution der DEAE-RSepharose-Säule:**
Von der gewaschenen DEAE-RSepharose wurde der F VIII:C mit einer Acetat-gepufferten und Lysinhaltigen $CaCl_2$-Lösung (0,3 mol/l) verdrängt und das Eluat, 195 ml, durch Zugabe von 1,46 g Saccharose auf eine Endkonzentration von 0,75 % gebracht.
- **Dialyse**
195 ml Eluat wurden gegen 20 l Dialysepuffer 16 Std. bei +4 °C dialysiert. Von diesem Material wurde der Eiweißgehalt gemessen ($OD_{280nm}$ = 10 mg/ml), die F VIII-Aktivität bestimmt und aus beiden Werten die Ausbeute und spezifische Aktivität berechnet; Teilmengen wurden in einem Provokationstest bei 20 °C gelagert und die F VIII-Aktivität nach 24, 48 und 96 Std. bestimmt (s. Tabelle, Versuch 1).
- **Einstellen der Konzentration und Sterilfiltration:** 218 ml dialysierter F VIII:C wurden über Sartorius-Filtern sterilfiltriert; die Aktivität wird auf 28 E/ml F VIII:C eingestellt, die Lösung abgefüllt und lyophilisiert.

Beispiel 2

- **Ausgangsmaterial:** Kryopräzipitat, 1 kg wurde mit 3 l Lösepuffer bei +37 °C gelöst und anschließend mit
- **2 x 5 % (v/v) $Al(OH)_3$** behandelt: 4 l Lösung werden mit jeweils 2 x 200 ml $Al(OH)_3$ 1 %ig 15 min bei 25°C adsorbiert, anschließend bei 3000 Upm abzentrifugiert und der Überstand für die Pasteurisierung mit Stabilisatoren versetzt.
- **Stabilisieren und Erhitzen:** Dazu wurden die 4040 ml des $Al(OH)_3$-Überstandes durch Zugabe von 4040 g Saccharose auf eine Endkonzentration entsprechend 100 % (w/v) gebracht; anschließend wurden 545,4 g Glycin entsprechend 1,8 mol/l zugesetzt und die $CaCl_2$-Konzentration auf 5 mmol/l eingestellt. Die stabilisierte Lösung wurde mit 2,5 N NaOH auf pH 7,3 eingestellt und dann 10 Std. bei +60 °C erhitzt. Vor und nach Erhitzen wurde die F VIII:C-Aktivität bestimmt, und zwar mit 2,0 E/ml bzw. 1,9 E/ml.
- **Vorbereitung zur DEAE-Adsorption** gemäß DE 34 32 083: Die erhitzte Kryolösung (6868 ml) wurde mit 6868 ml Verdünnungspuffer 1:2 verdünnt und mit 2 N Essigsäure auf pH 5,5 eingestellt.
- **Adsorption an DEAE-RSepharose CL-6B im Batchverfahren:** 13736 ml verdünnte Kryolösung wurden mit 300 ml äquilibrierter DEAE-RSepharose CL-6B 4 Std. bei Raumtemperatur adsorbiert und anschließend die Sepharose abgetrennt.
- **Waschen der RSepharose und Überführen in eine Säule:** Die mit F VIII beladene Sepharose wurde auf einer Nutsche vorgewaschen, anschließend in eine Saule (∅ 7,2 cm) überführt und mit 3 l Waschpuffer behandelt.
- **Elution der DEAE-RSepharose-Säule:**
Von der gewaschenen DEAE-RSepharose wurde der F VIII:C mit einer Acetat-gepufferten und Lysinhaltigen $CaCl_2$-Lösung (0,3 mol/l) verdrängt und das Eluat, 195 ml, durch Zugabe von 1,46 g Saccharose auf eine Endkonzentration von 0,75 % gebracht.
- **Dialyse**
195 ml Eluat wurden gegen 20 l Dialysepuffer 16 Std. bei +4 °C dialysiert. Von diesem Material wurde der Eiweißgehalt über die OD bei 280 nm gemessen, die F VIII-Aktivität bestimmt und aus beiden Werten die Ausbeute und spezifische Aktivität berechnet; Teilmengen wurden in einem Provokationstest bei 20 °C gelagert und die F VIII-Aktivität nach 24, 48 und 96 Std. bestimmt (s. Tabelle, Versuch 2).

6

- **Einstellen der Konzentration und Sterilfiltration:** 218 ml dialysierter F VIII:C wurden über Sartorius-Filtern sterilfiltriert, die Aktivität der Lösung mit Dialysepuffer auf 28 E/ml F VIII:C eingestellt, die Lösung abgefüllt und lyophilisiert.

Beispiel 3

Analog Beispiel 1 wurde eine Lösung von frischem Kryopräzipitat zunächst 1 x 5 % (v/v) $Al(OH)_3$ adsorbiert und der Überstand nach Zentrifugation anschließend mit einer zweiten Menge von 5 % (v/v) $Al(OH)_3$ adsorbiert, nach 5 min bei 25 °C wurde 3 % (w/v) feuchtes ECTEOLA (120 g/4 l) zugesetzt und 15 min inkubiert. Aus dem Überstand nach Abtrennung der Adsorptionsmittel wurde, wie bereits beschrieben, pasteurisierter, hochgereinigter Faktor VIII gewonnen. Wie der Versuch 3 in der Tabelle ausweist, kommt man auch so mit guter Ausbeute zu einem stabilen Produkt mit hoher spezifischer Aktivität.

Beispiel 4

Gemäß Beispiel 1 wurde wiederum 1 kg Kryopräzipitat bei 37 °C frisch gelost, aber anschließend nur **einmal** mit
- **5 % (v/v) $Al(OH)_3$ adsorbiert:** 4 l gelöstes Kryopräzipitat mit 200 ml $Al(OH)_3$, 1 %ig, bei +25 °C 15 min adsorbiert und anschließend das Adsorbens bei 3000 Upm abzentrifugiert. Anschließend erfolgte eine sogenannte Mischadsorption, nämlich mit:
- **1x5% (v/v) $Al(OH)_3$-Adsorption und 3% QAE-[R]Sephadex-A50:** Zunächst wurden die 4 l des ersten $Al(OH)_3$-Überstandes mit 200 ml $Al(OH)_3$, 1 %ig, versetzt und 5 min damit gerührt, bevor 120 g feuchtes QAE-Sephadex A50 zugesetzt und 15 min bei +25 °C damit adsorbiert wurden. Der nach Zentrifugation gewonnene Überstand wurde gemäß Beispiel 1 stabilisiert, pasteurisiert und über Adsorption an DEAE-[R]Sepharose gereinigt. Ausbeute, spezifische Aktivität und Stabilität enthält die Tabelle (siehe Versuch 4).

Beispiel 5

Wie in Beispiel 1 wurde 1 kg Kryopräzipitat mit 3,0 l Puffer gelöst und mit 200 ml 1%iger $Al(OH)_3$ -Lösung 15 min bei 25°C adsorbiert und der Überstand durch Zentrifugation gewonnen. Anschließend wurde er wie folgt behandelt:
- jeweils 1 x mit 5 % (v/v) $Al(OH)_3$, 3 % (w/v) feuchtem QAE-[R]Sephadex A50 und 3 % (w/v) feuchtem ECTEOLA; die Adsorptionsmittel wurden im Abstand von 5 min zugegeben, 15 min bei 25°C inkubiert und 15 min nach Zugabe von ECTEOLA abzentrifugiert (3000 Upm). Auch die Pasteurisierung und Hochreinigung erfolgte gemäß Beispiel 1. Das Endprodukt ist hinsichtlich Ausbeute und Eigenschaften unter Versuch 5 in der Tabelle charakterisiert.

Beispiel 6

Gemäß Beispiel 2 wurde 1 kg frisch gelöstes Kryopräzipitat eingesetzt und einmal mit 5 % (v/v) $Al(OH)_3$ adsorbiert und der Überstand dann wie folgt adsorbiert:
- 1 x mit 5 % (v/v) $Al(OH)_3$, dann mit 3 % QAE, abschließend mit 1 % $Ca_3(PO_4)_2$:
Zuerst wurden 4 l des ersten $Al(OH)_3$-Überstandes mit weiteren 200 ml $Al(OH)_3$, 1%ig, (5 min, 25°C), dann unter identischen Bedingungen mit 120 g QAE-[R]-Sephadex A50 und abschließend mit 40 g $Ca_3(PO_4)_2$ 15 min bei +25°C behandelt. Alle Adsorbentien wurden zusammen abzentrifugiert und der Überstand nach Zentrifugation mit Stabilisatoren versetzt, pasteurisiert, verdünnt und der F VIII über DEAE-Sepharose gemäß Beispiel 1 gereinigt. Das Eluat imponierte durch eine hohe spezifische Aktivität (siehe Versuch 6 in der Tabelle).
Auch Kryopräzipitate, wie sie gemäß EP 0 018 561 B1 oder EP 0 032 655 zu F VIII-Konzentraten aufgearbeitet werden, führen mit guter Ausbeute zu stabilen und hochwirksamen Produkten, wenn das Ausgangsmaterial erfindungsgemäß mit Adsorption behandelt wurde. Das zeigt das nachfolgende Beispiel.

Beispiel 7

6 kg Rohkryopräzipitat werden mit 18 l eines Puffers folgender Zusammensetzung bei 37°C gelost: 0,08 mol/l NaCl, 0,27 mol/l Glycin, 0,5 E/ml Heparin, 0,1 E/ml AT III, pH 6,0. Es wurden 24 l Rohkryolösung erhalten und zunächst mit
- 1 x 5 % (v/v) $Al(OH)_3$ adsorbiert:

Dazu wurden bei 25°C zu 24 l Rohkryolösung 1,2 l Al(OH)$_3$, 1 %ig, zugesetzt, 15 min gerührt und das Adsorbens bei 3000 Upm abzentrifugiert; anschließend erfolgte eine Mischadsorption mit:

- 5 % (v/v) Al(OH)$_3$ und 3 % QAE-$^R$Sephadex A50:

Zu 24 l des ersten Al(OH)$_3$-Überstandes wurden noch einmal 1,2 l Al(OH)$_3$, 1 %ig, zugegeben und 5 min gerührt; anschließend wurde die Lösung mit 720 g feuchtem QAE-$^R$Sephadex A50 weitere 15 min bei 25°C adsorbiert. Der durch Zentrifugation gewonnene Überstand wurde gemäß Ma 339 (EP 0 018 561; USP 4 297 344) zu F VIII-Konzentrat aufgearbeitet und lyophilisiert.

Das rekonstituierte Endprodukt imponierte durch gute Stabilität beim Stehen bei Zimmertemperatur:

```
F VIII:C-Aktivität nach Rekonstitution:            29 IE/ml
               nach 2 Std. bei 23°C in Lösung 27 IE/ml
               nach 6 Std. bei 23°C in Lösung 29 IE/ml
               nach 24Std. bei 23°C in Lösung 30 IE/ml.
```

In den Beispielen verwendete Puffer:

| | |
|---|---|
| Lösepuffer: | 0,08 mol/l NaCl |
| | 0,27 mol/l Glycin |
| | + 0,1 E/ml AT III |
| | + 0,5 E/ml Heparin |
| Verdünnungspuffer: | 0,2 mol/l Lysin |
| | 0,2 mol/l Na-Acetat |
| | pH 5,5 |
| Waschpuffer: | 0,1 mol/l Lysin |
| | 0,1 mol/l Na-Acetat |
| | 0,017 mol/l NaCl |
| | 0,0125 mol/l CaCl$_2$ |
| | pH 5,5 |
| Elutionspuffer: | 0,1 mol/l Lysin |
| | 0,1 mol/l Na-Acetat |
| | 0,3 mol/l CaCl$_2$ |
| | pH 5,5 |
| Dialyse-und Abfüllpuffer: | 0,15 mol/l NaCl |
| | 0,75 % Saccharose |
| | 3 % Glycin |
| | pH 6,9 |

**Patentansprüche**

1.  Verfahren zur Herstellung von Faktor VIII mit hoher spezifischer Aktivität und Stabilität, dadurch gekennzeichnet, daß man eine Faktor VIII-haltige Lösung zunächst mindestens zwei Adsorptionen an Al(OH)$_3$ und dann einer Adsorption an einem Anionenaustauscher und/ oder Ca$_3$(PO$_4$)$_2$ unterwirft, bevor man aus der so vorbehandelten Lösung mit an sich bekannten Methoden den Faktor VIII gewinnt.

2.  Verfahren nach Anspruch 1, dadurch gekennzeichnet, daß mit einem basischen Austauscher mit einer hydrophilen Matrix und anionischen Gruppierungen behandelt wird.

3.  Verfahren nach Anspruch 1, dadurch gekennzeichnet, daß mit einem basischen Anionenaustauscher mit einer Matrix auf Polysaccharidbasis und anionischen Gruppierungen behandelt wird.

4.  Verfahren nach Anspruch 1, dadurch gekennzeichnet, daß die Behandlung bei einem pH-Wert von 6,5 - 7,5 und einer Leitfähigkeit von 10 - 15 mS durchgeführt wird.

5.  Verfahren nach Anspruch 1, dadurch gekennzeichnet, daß die Behandlung in einem Medium durchgeführt wird, das keine Citrationen oder andere Fänger von zweiwertigen Metallionen enthält.

6. Verfahren nach Anspruch 1, dadurch gekennzeichnet, daß zwei verschiedene Adsorbentien nacheinander zugegeben und dann der Überstand abgetrennt wird.

7. Verfahren nach Anspruch 1, dadurch gekennzeichnet, daß zusätzlich pasteurisiert wird.

8. Verfahren nach Anspruch 1, dadurch gekennzeichnet, daß Calciumphosphat in einer Menge von 10 g oder ein feuchter Anionenaustauscher in einer Menge von 20-30 g/l Lösung verwendet werden.

## Claims

1. A process for the preparation of factor VIII with high specific activity and stability, which comprises subjecting a factor VIII-containing solution first to at least two adsorptions on $Al(OH)_3$ and then to an adsorption on an anion exchanger and/or $Ca_3(PO_4)_2$ before the factor VIII is obtained by methods known per se from the solution pretreated in this way.

2. The process as claimed in claim 1, wherein a basic exchanger with a hydrophilic matrix and anionic groups is used for the treatment.

3. The process as claimed in claim 1, wherein a basic anion exchanger with a polysaccharide-based matrix and anionic groups is used for the treatment.

4. The process as claimed in claim 1, wherein the treatment is carried out at a pH of 6.5-7.5 and at a conductivity of 10-15 mS.

5. The process as claimed in claim 1, wherein the treatment is carried out in a medium which contains no citrate ions or other traps for doubly charged metal ions.

6. The process as claimed in claim 1, wherein two different adsorbents are added successively and then the supernatant is separated off.

7. The process as claimed in claim 1, wherein pasteurization is additionally carried out.

8. The process as claimed in claim 1, wherein calcium phosphate is used in an amount of 10 g or a moist anion exchanger is used in an amount of 20-30 g/l of solution.

## Revendications

1. Procédé de préparation d'un facteur VIII d'activité spécifique élevée et de grande stabilité, caractérisé en ce qu'on soumet une solution contenant le facteur VIII d'abord à au moins deux absorptions sur $Al(OH)_3$ et ensuite à une adsorption sur un échangeur d'anions et/ou sur $Ca_3(PO_4)_2$, avant d'obtenir, à l'aide de techniques connues en soi, le facteur VIII à partir de la solution ainsi prétraitée.

2. Procédé selon la revendication 1, caractérisé en ce qu'on effectue le traitement avec un échangeur d'ions basique à matrice hydrophile et à groupements anioniques.

3. Procédé selon la revendication 1, caractérisé en ce qu'on effectue le traitement avec un échangeur d'anions basique à matrice à base de polysaccharide et à groupements anioniques.

4. Procédé selon la revendication 1, caractérisé en ce qu'on effectue le traitement à un pH de 6,5 à 7,5 et avec une conductivité de 10 à 15 mS.

5. Procédé selon la revendication 1, caractérisé en ce qu'on effectue le traitement dans un milieu qui ne contient pas d'ions citrate ou d'autres capteurs d'ions métalliques divalents.

6. Procédé selon la revendication 1, caractérisé en ce qu'on ajoute successivement deux adsorbants différents et qu'on sépare ensuite le surnageant.

7. Procédé selon la revendication 1, caractérisé en ce qu'on procède en outre à une pasteurisation.

8. Procédé selon la revendication 1, caractérisé en ce qu'on utilise du phosphate de calcium à raison de 10 g, ou un échangeur d'anions humidifié à raison de 20 à 30 g, par litre de solution.